(19) Europäisches Patentamt
European Patent Office
Office européen des brevets

(11) **EP 4 197 597 A1**

(12) **EUROPEAN PATENT APPLICATION**
published in accordance with Art. 153(4) EPC

(43) Date of publication:
**21.06.2023 Bulletin 2023/25**

(21) Application number: **21869173.1**

(22) Date of filing: **01.09.2021**

(51) International Patent Classification (IPC):
**A61Q 1/00** (2006.01)   **A61K 8/02** (2006.01)
**A61K 8/25** (2006.01)   **A61K 8/88** (2006.01)

(52) Cooperative Patent Classification (CPC):
**A61K 8/02; A61K 8/25; A61K 8/88; A61Q 1/00**

(86) International application number:
**PCT/JP2021/032136**

(87) International publication number:
**WO 2022/059486 (24.03.2022 Gazette 2022/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.09.2020   JP 2020154913**

(71) Applicant: **Mitsui Chemicals, Inc.**
**Tokyo 104-0028 (JP)**

(72) Inventors:
• **SHIMIZU, Akinori**
  **Sodegaura-shi, Chiba 299-0265 (JP)**
• **YOSHIMURA, Masafumi**
  **Sodegaura-shi, Chiba 299-0265 (JP)**
• **OGAWA, Takeshi**
  **Tokyo 124-0012 (JP)**

(74) Representative: **Mewburn Ellis LLP**
**Aurora Building**
**Counterslip**
**Bristol BS1 6BX (GB)**

(54) **POWDER COSMETIC AND COSMETIC COMPOSITION**

(57)   A powder cosmetic containing: an inorganic powder; and a polyaspartic acid alkali metal salt that coats a part or all of a surface of the inorganic powder, in which a weight average molecular weight x of the polyaspartic acid alkali metal salt is from 1,000 to 150,000, and the weight average molecular weight x of the polyaspartic acid alkali metal salt and a mass ratio y of the polyaspartic acid alkali metal salt to a total amount of the inorganic powder and the polyaspartic acid alkali metal salt satisfy the following relationships: $y \le 1.6 \times 10^7 x^{-2.275}$ and $y \ge 1.3 \times 10^5 x^{-2.232}$.

FIG.2

MASS RATIO OF POLYASPARTIC ACID ALKALI METAL SALT [-]

WEIGHT AVERAGE MOLECULAR WEIGHT OF POLYASPARTIC ACID ALKALI METAL SALT [-]

**Description**

Technical Field

The present disclosure relates to a powder cosmetic and a cosmetic composition.

Background Art

The powder cosmetic imparts characteristics such as preferable aesthetic appearance due to optical characteristics of powder. Conventionally, a powder cosmetic having a better sense of use has been developed by improving components, blending amount, or the like of powder.

For example, Japanese Patent Application Laid-Open (JP-A) No. 2006-265214 describes a powder cosmetic containing (a) a specific heteromorphic composite powder, and (b) one or two or more water-soluble components selected from amino acid, polyamino acid, a derivative or a salt thereof. JP-A No. 2006-265214 describes that the powder cosmetic is thus excellent in smooth spreadability, adhesion force to the skin and moisturizing feeling, and effect of making the unevenness of the skin inconspicuous at the time of applying the cosmetic.

SUMMARY OF INVENTION

Technical Problem

However, JP-A No. 2006-265214 is based on the premise that a heteromorphic composite powder in which a plurality of substantially spherical particles are aggregated and which has a plurality of irregularities on the surface thereof is used. In JP-A No. 2006-265214, confirmation of the smooth spreadability, adhesion force to the skin and moisturizing feeling, and effect of making the unevenness of the skin inconspicuous at the time of applying the cosmetic is performed only by sensory evaluation by panelists. That is, the effect is not confirmed by an objective index. Furthermore, JP-A No. 2006-265214 does not describe at all the influence in the case of overlaying the cosmetic (resistance feeling, occurrence of makeup creasing, and the like at the time of overlaying the cosmetic), and it has become clear that smooth spread of the cosmetic on the skin cannot be sufficiently obtained in some cases.

The disclosure has been made in view of the above, and an object of one aspect of the disclosure is to provide a powder cosmetic that has smooth spreadability with less resistance feeling to the skin and is less likely to cause makeup creasing at the time of overlaying the powder cosmetic. An object of another aspect of the disclosure is to provide a cosmetic composition that has smooth spreadability with less resistance feeling to the skin and is less likely to cause makeup creasing at the time of overlaying the cosmetic composition.

Solution to Problem

Specific means for solving the problem include the following aspects.

<1> A powder cosmetic containing:

an inorganic powder; and
a polyaspartic acid alkali metal salt that coats a part or all of a surface of the inorganic powder,
in which a weight average molecular weight x of the polyaspartic acid alkali metal salt is from 1,000 to 150,000, and
the weight average molecular weight x of the polyaspartic acid alkali metal salt and, a mass ratio y of the polyaspartic acid alkali metal salt to a total amount of the inorganic powder and the polyaspartic acid alkali metal salt satisfy the following Expressions (1) and (2):

$$y \leq 1.6 \times 10^7 x^{-2.275} ...\text{Expression (1)}$$

$$y \geq 1.3 \times 10^5 x^{-2.232} ...\text{Expression (2)}.$$

<2> The powder cosmetic according to <1>, in which the weight average molecular weight x of the polyaspartic acid alkali metal salt and the mass ratio y of the polyaspartic acid alkali metal salt to the total amount of the inorganic powder and the polyaspartic acid alkali metal salt further satisfy the following Expression (3):

$$y \leq 1.0 \times 10^7 x^{-2.275} \ldots \text{Expression (3)}.$$

<3> The powder cosmetic according to <1>, in which the weight average molecular weight x of the polyaspartic acid alkali metal salt and the mass ratio y of the polyaspartic acid alkali metal salt to the total amount of the inorganic powder and the polyaspartic acid alkali metal salt further satisfy the following Expression (4):

$$y \leq 8.0 \times 10^6 x^{-2.275} \ldots \text{Expression (4)}.$$

<4> The powder cosmetic according to any one of <1> to <3>, in which the weight average molecular weight x of the polyaspartic acid alkali metal salt and the mass ratio y of the polyaspartic acid alkali metal salt to the total amount of the inorganic powder and the polyaspartic acid alkali metal salt further satisfy the following Expression (5):

$$y \geq 1.5 \times 10^5 x^{-2.232} \ldots \text{Expression (5)}.$$

<5> The powder cosmetic according to any one of <1> to <4>, in which the weight average molecular weight x of the polyaspartic acid alkali metal salt is from 8,000 to 120,000.

<6> The powder cosmetic according to any one of <1> to <5>, in which the polyaspartic acid alkali metal salt is at least one selected from the group consisting of lithium polyaspartate, potassium polyaspartate, and sodium polyaspartate.

<7> The powder cosmetic according to any one of <1> to <6>, in which the polyaspartic acid alkali metal salt contains at least sodium polyaspartate.

<8> The powder cosmetic according to any one of <1> to <7>, in which the inorganic powder contains at least one selected from the group consisting of talc, mica, sericite, and titanium oxide.

<9> The powder cosmetic according to any one of <1> to <8>, in which an average particle diameter (D50) of the inorganic powder is from 1 $\mu$m to 100 $\mu$m.

<10> A cosmetic composition containing the powder cosmetic according to any one of <1> to <9> and at least one selected from water or an oil agent.

Advantageous Effects of Invention

According to one aspect of the disclosure, it is possible to provide a powder cosmetic that has smooth spreadability with less resistance feeling to the skin and is less likely to cause makeup creasing at the time of overlaying the powder cosmetic. According to another aspect of the disclosure, it is possible to provide a cosmetic composition that has smooth spreadability with less resistance feeling to the skin and is less likely to cause makeup creasing at the time of overlaying the cosmetic composition.

BRIEF DESCRIPTION OF DRAWINGS

Fig. 1 is a graph for explaining how to obtain a yield locus and a degree of curve.

Fig. 2 is a scatter plot showing the relationship of the relative value of the degree of curve with respect to the weight average molecular weight [-] of the polyaspartic acid alkali metal salt and the mass ratio [-] of the polyaspartic acid alkali metal salt.

DESCRIPTION OF EMBODIMENTS

Hereinafter, the contents of the disclosure will be described in detail. The description of the constituent elements described below may be made based on a representative embodiment of the disclosure, but the disclosure is not limited to such an embodiment.

In numerical value ranges described in the disclosure in a stepwise manner, an upper limit value or a lower limit value described in one numerical value range may be replaced with an upper limit value or a lower limit value of a numerical value range of other stepwise description. In addition, in the numerical value ranges described in the disclosure, an upper limit value or a lower limit value of the numerical value range may be replaced with values shown in examples.

The numerical ranges indicated with the use of the term "to" in the disclosure indicate ranges including the numerical values before and after the term "to" as the lower limit value and the upper limit value.

Furthermore, in the disclosure, the amount of each component in a composition such as a powder cosmetic or a cosmetic composition means the total amount of a plurality of corresponding substances present in the composition unless otherwise specified in a case in which a plurality of substances corresponding to each component in the composition are present.

In addition, in the notation of the group (atomic group) in the present specification, the notation not indicating substitution or unsubstitution includes those having a substituent as well as those not having a substituent.

The term "step" in the present specification encompasses therein not only independent steps, but also more steps as long as desired actions of the steps are achieved even when the steps are not able to be clearly distinguished from the other steps.

In the disclosure, "mass%" and "wt%" have the same meaning, and "parts by mass" and "parts by weight" have the same meaning.

Furthermore, in the disclosure, any combination of two or more preferred embodiments is a more preferred embodiment.

<<Powder Cosmetic>>

A powder cosmetic according to the disclosure is a powder cosmetic containing: an inorganic powder; and a polyaspartic acid alkali metal salt that coats a part or all of a surface of the inorganic powder, in which a weight average molecular weight x of the polyaspartic acid alkali metal salt is from 1,000 to 150,000, and the weight average molecular weight x of the polyaspartic acid alkali metal salt and a mass ratio y of the polyaspartic acid alkali metal salt to a total amount of the inorganic powder and the polyaspartic acid alkali metal salt satisfy the following Expressions (1) and (2):

$$y \leq 1.6 \times 10^7 x^{-2.275} ...\text{Expression (1)}$$

$$y \geq 1.3 \times 10^5 x^{-2.232} ...\text{Expression (2)}.$$

The powder cosmetic of the disclosure has smooth spreadability with less resistance feeling to the skin and is less likely to cause makeup creasing at the time of overlaying the powder cosmetic.

The action of the powder cosmetic according to the disclosure is not clear, but is estimated as follows.

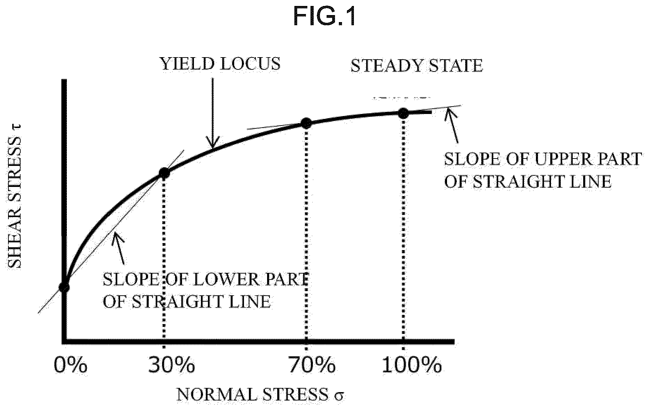

In the disclosure, powder bed shear force measurement is used for evaluation of the powder cosmetic at the time of overlaying the powder cosmetic. In the powder bed shear force measurement, a load is applied in the vertical direction and the horizontal direction to a powder filled in cells, and the normal stress $\sigma$ is attenuated after the normal stress $\sigma$ and the shear stress $\tau$ become a steady state, thereby obtaining a yield locus representing a relationship of the shear stress $\tau$ with respect to the normal stress $\sigma$ (Fig. 1). Such a state of the powder bed when being sheared while a load is applied is similar to a state of the powder bed when the powder cosmetic is further overlaid (for example, touch-up, concealment of spots, or concealment of stain) on the skin to which the powder cosmetic has been attached with a cosmetic tool to which the powder cosmetic has been attached. Therefore, in the disclosure, objective evaluation is performed by adopting an evaluation method using powder bed shear force measurement as a method of evaluating a sense of use when the powder cosmetic is overlaid.

Although the effect of the present application is most exhibited at the time of overlaying the powder cosmetic, the same effect is naturally expected even when the powder cosmetic is not overlaid. That is, to exhibit the effect of the present application, the effect of the present application (that is, to provide a powder cosmetic that has smooth spreadability with less resistance feeling to the skin and is less likely to cause makeup creasing) is exhibited not only in a case in which the powder cosmetic according to the disclosure is overlaid on the powder cosmetic according to the disclosure, but also in a case in which the powder cosmetic according to the disclosure is applied to the skin, in a case in which the powder cosmetic according to the disclosure is applied onto primer (liquid, cream, etc.) that has been applied to the skin, or in a case in which the powder cosmetic according to the disclosure is applied to blusher, concealer, or the like (liquid, powder, etc.) that has been applied onto the powder cosmetic according to the disclosure.

In the disclosure, the term "sense of use" of the powder cosmetic, the cosmetic composition, or the like includes a sensation of being smoothly spread with less resistance feeling to the skin and being less likely to cause makeup creasing at the time of overlaying the powder cosmetic and at the time of simply applying the powder cosmetic, or the like. In the disclosure, in order to directly refer to spreadability or makeup creasing, the spreadability or makeup creasing may be described in parallel with the sense of use. In the disclosure, a sensation felt by the user, such as skin compatibility, moisture feeling, or smoothness, of the powder cosmetic or the cosmetic composition is also a type of "sense of use" of the powder cosmetic, the cosmetic composition, or the like. The makeup creasing means that the powder bed attached to the skin moves, and refers to, for example, foundation creasing.

As shown in Fig. 1, the degree of curve can be obtained from a yield locus obtained by the powder bed shear force measurement. The degree of curve is calculated from the following equation: degree of curve = [slope of lower part of straight line of yield locus]/[slope of upper part of straight line of yield locus]. Note that the slope of the lower part of the straight line of the yield locus indicates the slope of a straight line (that is, a straight line connecting a plot when the value of the normal stress $\sigma$ is 0% and a plot when the value of the normal stress $\sigma$ is 30%) of the yield locus when the value of the normal stress $\sigma$ is from 0% to 30%, where the normal stress $\sigma$ at which the normal stress $\sigma$ and the shear stress $\tau$ are in a steady state is 100%. The slope of the upper part of the straight line of the yield locus indicates the slope of a straight line (that is, a straight line connecting a plot when the value of the normal stress $\sigma$ is 70% and a plot when the value of the normal stress $\sigma$ is 100%) of the yield locus when the value of the normal stress $\sigma$ is from 70% to 100%, where the normal stress $\sigma$ at which the normal stress $\sigma$ and the shear stress $\tau$ are in a steady state is 100%. The degree of curve correlates with the smoothness of the powder. As the numerical value of the degree of curve is high, shear adhesion stress is high, and the smoothness of the powder is low, that is, a problem that the powder cosmetic does not have smooth spreadability with less resistance feeling to the skin and is likely to cause makeup creasing at the time of overlaying the powder cosmetic tends to occur. On the other hand, as the numerical value of the degree of curve is low, shear adhesion stress is low, and the smoothness of the powder is high, that is, the above-described problem is less likely to occur at the time of overlaying the powder cosmetic. The term "shear stress $\tau$" is parallel to a certain cross section inside the powder bed, and indicates a stress required when a shearing operation is performed by applying a pair of forces opposite to each other to the upper and lower beds of the cross section, thereby causing the powder beds to slide along the surface. That is, the shear stress $\tau$ is a stress that causes the upper and lower powder beds to slide on a certain surface in a direction parallel to the surface in the powder bed. The term "shear adhesion stress" is a stress that is equal to a stress of the intercept at which a straight line obtained by approximating the yield locus by a least-squares method intersects the axis of the shear stress $\tau$. That is, the shear adhesion stress indicates an extrapolated value of the shear stress $\tau$ when the normal stress $\sigma$ of the approximate expression reaches 0. The shear stress $\tau$ and the shear adhesion stress are used as indices of the adhesion force between compacted powders.

The powder cosmetic according to the disclosure contains an inorganic powder and a polyaspartic acid alkali metal salt. The presence of the polyaspartic acid alkali metal salt between the inorganic powders (that is, the surfaces of particles when particles of the inorganic powder are close to each other) reduces the frictional force between the inorganic powders, thus making it possible to improve the smoothness of the powder cosmetic. The significance of Expressions (1) and (2) according to the disclosure, satisfied by the weight average molecular weight x of the polyaspartic acid alkali metal salt and the mass ratio y of the polyaspartic acid alkali metal salt to the total amount of the inorganic powder and the polyaspartic acid alkali metal salt, is estimated as follows.

In the powder cosmetic according to the disclosure, when the weight average molecular weight x of the polyaspartic acid alkali metal salt is excessively large, the interaction between the polyaspartic acid alkali metal salts (that is, the interaction between molecules of the polyaspartic acid alkali metal salt) is intensive. As a result, an increase in viscosity becomes significant, and the production suitability such as handling is impaired. Furthermore, the following problems may easily occur. At this time, the numerical value of the degree of curve of the powder cosmetic is high.

(1a) The friction between powder beds of the powder cosmetic is large, and smoothness is low.
(1b) The powder cosmetic does not have smooth spreadability with less resistance feeling to the skin and is likely to cause makeup creasing at the time of overlaying the powder cosmetic.

In the powder cosmetic according to the disclosure, when the mass ratio y of the polyaspartic acid alkali metal salt to the total amount of the inorganic powder and the polyaspartic acid alkali metal salt is excessively large, the following problems occur. At this time, the numerical value of the degree of curve of the powder cosmetic is high.

(2a) Since the amount of the polyaspartic acid alkali metal salt present between the inorganic powders is large, the interaction between the polyaspartic acid alkali metal salts is intensive.
(2b) An increase in viscosity of a powder cosmetic containing an aqueous solution of the polyaspartic acid alkali metal salt is significant.
(2c) The friction between powder beds of the powder cosmetic is large, and smoothness is low.
(2d) The powder cosmetic does not have smooth spreadability with less resistance feeling to the skin and is likely to cause makeup creasing at the time of overlaying the powder cosmetic.

From the above, in the powder cosmetic, when the weight average molecular weight x of the polyaspartic acid alkali metal salt is excessively large and the mass ratio y of the polyaspartic acid alkali metal salt to the total amount of the inorganic powder and the polyaspartic acid alkali metal salt is excessively large, the numerical value of the degree of curve of the powder cosmetic becomes higher, and the above-described problems are more likely to occur.

On the other hand, in the powder cosmetic according to the disclosure, when the weight average molecular weight x of the polyaspartic acid alkali metal salt is excessively small, the interaction between polyaspartic acid alkali metal salts is weak, and the production suitability such as handling is impaired. Furthermore, the following problems may easily occur. Also in this case, the numerical value of the degree of curve of the powder cosmetic is high.

(3a) The friction between powder beds of the powder cosmetic due to the polyaspartic acid alkali metal salt is large, and the effect of improving smoothness cannot be sufficiently exhibited.
(3b) The powder cosmetic does not have smooth spreadability with less resistance feeling to the skin and is likely to cause makeup creasing at the time of overlaying the powder cosmetic.

In the powder cosmetic according to the disclosure, when the mass ratio y of the polyaspartic acid alkali metal salt to the total amount of the inorganic powder and the polyaspartic acid alkali metal salt is excessively small, the following problems occur. Also in this case, the numerical value of the degree of curve of the powder cosmetic is high.

(4a) Since the amount of the polyaspartic acid alkali metal salt present between the inorganic powders is small, the interaction between the polyaspartic acid alkali metal salts is weak.
(4b) Since the polyaspartic acid alkali metal salt is locally present on the inorganic powder, uniform flow of the powder cosmetic by the polyaspartic acid alkali metal salt is inhibited, and the friction between powder beds is large, and the effect of improving smoothness cannot be sufficiently exhibited.
(4c) The powder cosmetic does not have smooth spreadability with less resistance feeling to the skin and is likely to cause makeup creasing at the time of overlaying the powder cosmetic.

From the above, in the powder cosmetic, when the weight average molecular weight x of the polyaspartic acid alkali metal salt is excessively small and the mass ratio y of the polyaspartic acid alkali metal salt to the total amount of the inorganic powder and the polyaspartic acid alkali metal salt is excessively small, the numerical value of the degree of curve of the powder cosmetic also becomes higher, and the above-described problems are more likely to occur.

From the above-described reasons, in the powder cosmetic, Expressions (1) and (2) satisfied by the weight average molecular weight x of the polyaspartic acid alkali metal salt and the mass ratio y of the polyaspartic acid alkali metal salt to the total amount of the inorganic powder and the polyaspartic acid alkali metal salt were determined. In the disclosure, the fact that the weight average molecular weight x of the polyaspartic acid alkali metal salt is excessively large means that the weight average molecular weight of the polyaspartic acid alkali metal salt is more than 150,000. The fact that the weight average molecular weight x of the polyaspartic acid alkali metal salt is excessively small means that the weight average molecular weight of the polyaspartic acid alkali metal salt is less than 1,000.

As described above, the coefficients in Expression (1) "$y \leq 1.6 \times 10^7 x^{-2.275}$" and Expression (2) "$y \geq 1.3 \times 10^5 x^{-2.232}$" satisfied by the weight average molecular weight x of the polyaspartic acid alkali metal salt and the mass ratio y of the polyaspartic acid alkali metal salt to the total amount of the inorganic powder and the polyaspartic acid alkali metal salt are numerical values derived from experience.

In the powder cosmetic according to the disclosure, the value of the mass ratio of the polyaspartic acid alkali metal salt to the total amount of the inorganic powder and the polyaspartic acid alkali metal salt is controlled to be a relatively small value. Therefore, although the polyaspartic acid alkali metal salt coats a part or all of the surface of the particles of the inorganic powder, the coating amount is suppressed to be relatively small. Therefore, in the powder cosmetic according to the disclosure, an excessive increase in viscosity caused by the polyaspartic acid alkali metal salt which is hydrophilic is suppressed.

In the disclosure, the mass ratio of the polyaspartic acid alkali metal salt to the total amount of the inorganic powder and the polyaspartic acid alkali metal salt is also simply referred to as "mass ratio of the polyaspartic acid alkali metal salt".

The value of the mass ratio of the polyaspartic acid alkali metal salt to the total amount of the inorganic powder and the polyaspartic acid alkali metal salt according to the disclosure is dimensionless, and thus does not have a unit.

Regarding the weight average molecular weight x of the polyaspartic acid alkali metal salt and the mass ratio y of the polyaspartic acid alkali metal salt to the total amount of the inorganic powder and the polyaspartic acid alkali metal salt, the powder cosmetic of the disclosure exhibits effects in a region surrounded by a range where $y \leq 1.6 \times 10^7 x^{-2.275}$ (Expression (1)) and $y \geq 1.3 \times 10^5 x^{-2.232}$ (Expression (2)) and the weight average molecular weight x of the polyaspartic acid alkali metal salt is from 1,000 to 150,000.

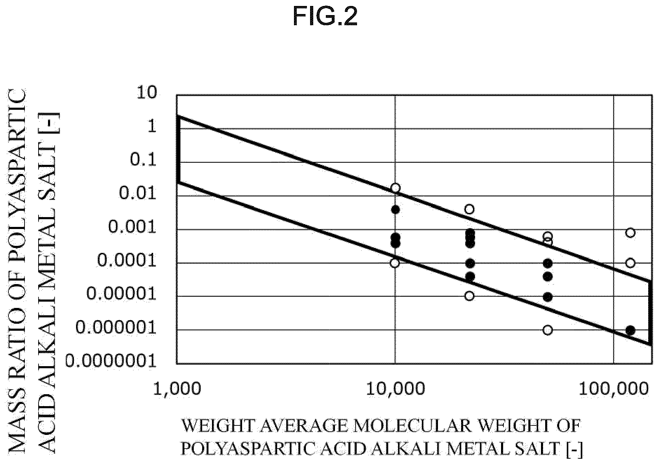

The range corresponds to the range indicated by the bold quadrilateral in the graph area of Fig. 2.

Regarding the weight average molecular weight x of the polyaspartic acid alkali metal salt and the mass ratio y of the polyaspartic acid alkali metal salt to the total amount of the inorganic powder and the polyaspartic acid alkali metal salt, the powder cosmetic of the disclosure exhibits more effects in a region surrounded by a range where $y \leq 1.0 \times 10^7 x^{-2.275}$ (Expression (3)) and $y \geq 1.3 \times 10^5 x^{-2.232}$ (Expression (2)), and the weight average molecular weight x of the polyaspartic acid alkali metal salt is from 1,000 to 150,000.

Regarding the weight average molecular weight x of the polyaspartic acid alkali metal salt and the mass ratio y of the polyaspartic acid alkali metal salt to the total amount of the inorganic powder and the polyaspartic acid alkali metal salt, the powder cosmetic of the disclosure exhibits more effects in a region surrounded by a range where $y \leq 8.0 \times 10^6 x^{-2.275}$ (Expression (4)) and $y \geq 1.3 \times 10^5 x^{-2.232}$ (Expression (2)), and the weight average molecular weight x of the polyaspartic acid alkali metal salt is from 1,000 to 150,000.

Regarding the weight average molecular weight x of the polyaspartic acid alkali metal salt and the mass ratio y of the polyaspartic acid alkali metal salt to the total amount of the inorganic powder and the polyaspartic acid alkali metal salt, the powder cosmetic of the disclosure exhibits more effects in a region surrounded by a range where $y \leq 1.6 \times 10^7 x^{-2.275}$ (Expression (1)) and $y \geq 1.5 \times 10^5 x^{-2.232}$ (Expression (5)), and the weight average molecular weight x of the polyaspartic acid alkali metal salt is from 1,000 to 150,000.

Regarding the weight average molecular weight x of the polyaspartic acid alkali metal salt and the mass ratio y of the polyaspartic acid alkali metal salt to the total amount of the inorganic powder and the polyaspartic acid alkali metal salt, the powder cosmetic of the disclosure exhibits more effects in a region surrounded by a range where $y \leq 1.0 \times 10^7 x^{-2.275}$ (Expression (3)) and $y \geq 1.5 \times 10^5 x^{-2.232}$ (Expression (5)), and the weight average molecular weight x of the polyaspartic acid alkali metal salt is from 1,000 to 150,000.

Regarding the weight average molecular weight x of the polyaspartic acid alkali metal salt and the mass ratio y of the polyaspartic acid alkali metal salt to the total amount of the inorganic powder and the polyaspartic acid alkali metal salt, the powder cosmetic of the disclosure exhibits more effects in a region surrounded by a range where $y \leq 8.0 \times 10^6 x^{-2.275}$ (Expression (4)) and $y \geq 1.5 \times 10^5 x^{-2.232}$ (Expression (5)), and the weight average molecular weight x of the polyaspartic acid alkali metal salt is from 1,000 to 150,000.

From the viewpoint of enhancing the smoothness of the powder and the viewpoint of handling in the production described later, the powder cosmetic of the disclosure exhibits more effects in a region surrounded by a range where the above-described expressions are satisfied and the weight average molecular weight x of the polyaspartic acid alkali metal salt is from 8,000 to 120,000.

The powder cosmetic of the disclosure exhibits more effects in a region surrounded by a range of $y \leq 5.0 \times 10^6 x^{-2.275}$ and Expression (2) or (5), and the above-described range of the weight average molecular weight x of the polyaspartic acid alkali metal salt, from the viewpoint of having smooth spreadability with less resistance feeling to the skin and suppressing occurrence of makeup creasing.

The powder cosmetic of the disclosure exhibits more effects in a region surrounded by a range of Expression (1), Expression (3), or Expression (4) and $y \geq 2.5 \times 10^5 x^{-2.232}$, and the above-described range of the weight average molecular weight x of the polyaspartic acid alkali metal salt, from the viewpoint of having smooth spreadability with less resistance feeling to the skin and suppressing occurrence of makeup creasing. It is more preferable to replace the range of $y \geq 2.5 \times 10^5 x^{-2.232}$ with a range of $y \geq 4.0 \times 10^5 x^{-2.232}$, $y > 5.0 \times 10^5 x^{-2.232}$, or $y \geq 9.0 \times 10^5 x^{-2.232}$, from the viewpoint of having smooth spreadability with less resistance feeling to the skin and suppressing occurrence of makeup creasing.

When the weight average molecular weight x of the polyaspartic acid alkali metal salt and the mass ratio y of the polyaspartic acid alkali metal salt to the total amount of the inorganic powder and the polyaspartic acid alkali metal salt are values in a region surrounded by the above-described range, the interaction between the polyaspartic acid alkali metal salts is controlled to be not too intensive. As a result, the friction between powder beds can be reduced to increase the smoothness of the powder, handling in the production is improved, and thus the effect of the present application can be further obtained. The interaction between the polyaspartic acid alkali metal salts is also controlled to be not too weak. As a result, the friction between the powder beds is reduced to increase the smoothness of the powder, handling in the production is improved, and thus the effect of the present application can be further obtained.

As described above, there is a preferred range of the mass ratio y of the polyaspartic acid alkali metal salt with respect to the weight average molecular weight x of the polyaspartic acid alkali metal salt. Preferably, as the weight average molecular weight x of the polyaspartic acid alkali metal salt is larger, the mass ratio y of the polyaspartic acid alkali metal salt is smaller. Preferably, as the weight average molecular weight x of the polyaspartic acid alkali metal salt is smaller, the mass ratio y of the polyaspartic acid alkali metal salt is larger. The intensity of the interaction force between the polyaspartic acid alkali metal salts as a polymer varies depending on the magnitude of the weight average molecular weight x (or number average molecular weight) of the polyaspartic acid alkali metal salt. Therefore, it is considered that there is a preferable range of the mass ratio y of the polyaspartic acid alkali metal salt with respect to the weight average molecular weight x (or number average molecular weight) of the polyaspartic acid alkali metal salt.

Note that the disclosure is not limited to the above-described estimation mechanism at all.

(Polyaspartic Acid Alkali Metal Salt)

- Polyaspartic Acid Alkali Metal Salt -

The polyaspartic acid alkali metal salt is a polymer formed by an amide bond of an aspartic acid alkali metal salt, and generally has a structure including a plurality of the following structural units.

In the formula, n is an integer of 1 or more. In the formula, M represents an alkali metal. The alkali metal in M includes lithium, sodium, potassium, rubidium, cesium, or francium. The amide bond in the main chain of the polyaspartic acid alkali metal salt may be an $\alpha$-bond or a $\beta$-bond. These bonding forms may be the same or different for each structural unit. The amide bond is an $\alpha$-bond in a case in which the amino group of an adjacent structural unit is bonded to the carboxyl group at the $\alpha$-position of the aspartic acid alkali metal salt. The amide bond is a $\beta$-bond in a case in which the amino group of an adjacent structural unit is bonded to the carboxyl group at the $\beta$-position of the aspartic acid alkali metal salt.

The steric structures of the structural units may be each independently a L-form or a D-form, and may be, for example, a L-form polymer as a whole, a D-form polymer, or a racemate.

The configuration of the polyaspartic acid alkali metal salt according to the disclosure is not particularly limited as long as the powder cosmetic of the disclosure exhibits an effect. The polyaspartic acid alkali metal salt according to the disclosure preferably contains at least one polyaspartic acid alkali metal salt selected from the group consisting of lithium polyaspartate, potassium polyaspartate, and sodium polyaspartate. In one aspect of the disclosure, use of the polyaspartic acid alkali metal salt makes it possible to realize a powder cosmetic that has smooth spreadability with less resistance feeling to the skin and is less likely to cause makeup creasing at the time of overlaying the powder cosmetic. The polyaspartic acid alkali metal salt according to the disclosure more preferably contains at least sodium polyaspartate.

Sodium polyaspartate, which is an example of the polyaspartic acid alkali metal salt according to the disclosure, is a polymer formed by an amide bond of sodium aspartate, and generally has a structure including a plurality of the following structural units.

In the formula, n is an integer of 1 or more. The amide bond in the main chain of sodium polyaspartate may be an $\alpha$-bond or a $\beta$-bond. These bonding forms may be the same or different for each structural unit. The amide bond is an $\alpha$-bond in a case in which the amino group of an adjacent structural unit is bonded to the carboxyl group at the $\alpha$-position of sodium aspartate. The amide bond is a $\beta$-bond in a case in which the amino group of an adjacent structural unit is bonded to the carboxyl group at the $\beta$-position of sodium aspartate.

- Molecular Weight of Polyaspartic Acid Alkali Metal Salt -

Hereinafter, the molecular weight of the polyaspartic acid alkali metal salt will be described in detail, from the viewpoint of the weight average molecular weight (Mw), the number average molecular weight (Mn), and the polydispersity of the molecular weight (weight average molecular weight (Mw)/number average molecular weight (Mn)).

The problems that occur when the weight average molecular weight of the polyaspartic acid alkali metal salt is excessively large are as described above. When the viscosity of the aqueous solution of the polyaspartic acid alkali metal salt is remarkably increased, stringiness is expressed. Expression of stringiness makes handling at the time of powder mixing in the production process of the powder cosmetic difficult. The viscosity can be suppressed by further diluting the aqueous solution of the polyaspartic acid alkali metal salt with water, but the addition amount of water with respect to the inorganic powder increases. As a result, the powder absorbs moisture at the time of powder mixing, and adhesion to the mixing tank and aggregation between powders occur, resulting in difficulty in uniform powder mixing. In order to solve such a difficulty at the time of powder mixing, it is also conceivable to previously concentrate the aqueous solution of the polyaspartic acid alkali metal salt to thereby reduce the addition amount of concentrated aqueous solution of the polyaspartic acid alkali metal salt with respect to the inorganic powder. However, since it is necessary to newly introduce a concentration process in the production process of the powder cosmetic, the production cost increases.

The problems that occur when the weight average molecular weight of the polyaspartic acid alkali metal salt is excessively small are as described above.

From the viewpoints described above, the weight average molecular weight of the polyaspartic acid alkali metal salt according to the disclosure is from 1,000 to 150,000.

The weight average molecular weight of the polyaspartic acid alkali metal salt according to the disclosure is from 1,000 to 150,000. The weight average molecular weight of the polyaspartic acid alkali metal salt according to the disclosure is not particularly limited, but is preferably from 8,000 to 120,000, more preferably from 10,000 to 120,000, still more preferably from 10,000 to 100,000, particularly preferably from 16,000 to 60,000, even more preferably from 20,000 to 35,000, and still even more preferably from 20,000 to 27,000, from the viewpoint of enhancing the smoothness of the powder, handling in the production, and the like.

In the polyaspartic acid alkali metal salt according to the disclosure, the number average molecular weight of the polyaspartic acid alkali metal salt is not particularly limited, and may be, for example, from 1,000 to 60,000. Incidentally, the number average molecular weight of the polyaspartic acid alkali metal salt is preferably from 6,000 to 50,000, more preferably from 7,000 to 48,000, still more preferably from 8,000 to 40,000, particularly preferably from 10,000 to 36,000, even more preferably from 12,000 to 20,000, and still even more preferably from 13,000 to 16,000, from the viewpoint of enhancing the smoothness of the powder, handling in the production, and the like.

The polydispersity of the molecular weight of the polyaspartic acid alkali metal salt according to the disclosure is not particularly limited, and may be, for example, from 1.00 to 2.60. The polydispersity of the molecular weight of the polyaspartic acid alkali metal salt is preferably from 1.20 to 2.40, more preferably from 1.30 to 1.90, still more preferably from 1.40 to 1.80, particularly preferably from 1.50 to 1.75, and extremely preferably from 1.50 to 1.70, from the viewpoint of enhancing the smoothness of the powder, handling in the production, and the like.

The polyaspartic acid alkali metal salt having a weight average molecular weight, a number average molecular weight, and a polydispersity of the molecular weight within the above-described ranges can be obtained, for example, by using a method of producing a polyaspartic acid alkali metal salt described later.

- Method of Measuring Molecular Weight of Polyaspartic Acid Alkali Metal Salt -

In the disclosure, the weight average molecular weight and the number average molecular weight of the polyaspartic acid alkali metal salt each refer to a value measured by the following GPC measurement using gel permeation chromatography (GPC).

LC-Solution (manufactured by Shimadzu Corporation) as an analyzer,
RID-10A (manufactured by Shimadzu Corporation) as a detector,
DGU-20A (manufactured by Shimadzu Corporation) as a degasser,
LC-20AD (manufactured by Shimadzu Corporation) as a pump,
SIL-20A (manufactured by Shimadzu Corporation) as an automatic sampler,
CTO-20A (manufactured by Shimadzu Corporation) as a liquid feeding unit,
Shodex Asahipak GF-7M HQ $\times$ 1 (manufactured by Showa Denko K.K.) as a column, and
Shodex Asahipak GF-1G7B (manufactured by Showa Denko K.K.) as a guard column
are used. The temperature of the column oven is 45°C. The authentic sample is pullulan (Shodex STANDARD P-82 (authentic sample group kit, manufactured by Showa Denko K.K.)). Cubic calibration curves are prepared using P-5, P-10, P-20, P-50, P-100, and P-200 of the authentic samples. As the mobile phase, 0.1 mol/L saline is used. In the obtained elution curve, the weight average molecular weight and the number average molecular weight are calculated from the main peak of the polymer.

- Method of Producing Polyaspartic Acid Alkali Metal Salt -

As described in Japanese Patent Publication (JP-B) No. 3384420, the polyaspartic acid alkali metal salt may also be obtained by, for example, a method including: heating aspartic acid to 200°C to 230°C in a water-insoluble solvent to obtain a polysuccinimide; and hydrolyzing the obtained polysuccinimide in an aqueous solution of an alkali metal hydroxide salt to produce a polyaspartic acid alkali metal salt. As the water-insoluble solvent, a solvent that forms separated layers when mixed with water and that has a boiling point of 200°C or higher can be used. Examples of such a solvent include saturated hydrocarbon compounds such as n-paraffin and liquid paraffin, silicone-based oils, and fluorine-based oils. Among them, a solvent having a boiling point of 200°C or higher (preferably 230°C or higher) and a viscosity at 25°C of 100 cP or less (preferably, 20 cP or less) can be suitably used.

The polysuccinimide as a precursor can also be obtained by heating maleamic acid at a temperature of from 160°C to 330°C. Polysuccinimide can also be obtained by reacting maleic anhydride with aqueous ammonia in an aqueous solvent, and then heating the reaction solution to a temperature of at least 170°C. Alternatively, as described in International Publication No. 2011/102293, the polyaspartic acid alkali metal salt can also be produced by a method including: preparing a polyaspartic acid precursor polymer by performing polymerization using, as a monomer, at least one selected from a reactant of maleic anhydride and ammonia, or maleamic acid; and treating the obtained polyaspartic acid precursor polymer with an aqueous solution of an alkali metal hydroxide salt to obtain a polyaspartic acid alkali metal salt.

Among the polyaspartic acid alkali metal salts, for example, in an example of a method of producing sodium polyaspartate, the following procedures are performed: (1) L-aspartic acid is spread on a SUS tray, and the tray is allowed to stand in an oven at 230°C for 4 hours under normal pressure in a nitrogen atmosphere to obtain a powdery polysuccinimide; and (2) distilled water is further added to the obtained powdery polysuccinimide, a 32 mass% aqueous solution of sodium hydroxide is added dropwise little by little while the temperature of the solution is controlled to be from 45°C to 55°C, when the reactant acquires fluidity, pH measurement is started, the 32 mass% aqueous solution of sodium hydroxide is further added dropwise while the pH is measured, and when the pH falls within a range of from 10 to 10.5, the dropwise addition is terminated to obtain an aqueous sodium polyaspartate solution containing sodium polyaspartate.

Among the polyaspartic acid alkali metal salts, for example, in a case in which sodium polyaspartate is obtained by hydrolysis of polysuccinimide, succinimide structural units that have not been hydrolyzed may partially remain in the polymer. In other words, the sodium polyaspartate according to the disclosure may partially (for example, 10% or less, or 5% or less, or 1% or less of the total number of structural units) contain the succinimide structural unit or need not contain the succinimide structural unit at all.

- Mass Ratio of Polyaspartic Acid Alkali Metal Salt -

In the powder cosmetic according to the disclosure, the mass ratio of the polyaspartic acid alkali metal salt to the total amount of the inorganic powder and the polyaspartic acid alkali metal salt is preferably within a range of from 0.00000095 to 0.0045, and more preferably within a range of from 0.000035 to 0.0010. Within the above-described range, the powder cosmetic has even more smooth spreadability with less resistance feeling to the skin and is less likely to cause makeup creasing at the time of overlaying the powder cosmetic. The mass ratio of the polyaspartic acid alkali metal salt to the total amount of the inorganic powder and the polyaspartic acid alkali metal salt can be determined from the formula of [mass of polyaspartic acid alkali metal salt]/([mass of inorganic powder] + [mass of polyaspartic acid alkali metal salt]).

More specific description will be given as follows. In the powder cosmetic according to the disclosure, the polyaspartic acid alkali metal salt coats a part or all of the surface of the inorganic powder by mixing the polyaspartic acid alkali metal salt with the inorganic powder. The presence of the polyaspartic acid alkali metal salt between the inorganic powders reduces the frictional force between the inorganic powders, and thus allows the smoothness of the powder cosmetic to be improved.

Therefore, when the mass ratio of the polyaspartic acid alkali metal salt to the total amount of the inorganic powder and the polyaspartic acid alkali metal salt is excessively large, the problems (2a) to (2d) occur, that is, the problem at the time of overlaying the powder cosmetic occurs. On the other hand, when the mass ratio of the polyaspartic acid alkali metal salt to the total amount of the inorganic powder and the polyaspartic acid alkali metal salt is excessively small, the problems (4a) to (4c) occur, that is, the problem at the time of overlaying the powder cosmetic occurs.

The mass ratio of the polyaspartic acid alkali metal salt to the total amount of the inorganic powder and the polyaspartic acid alkali metal salt is preferably from 0.000035 to 0.0010, more preferably from 0.000035 to 0.00065, still more preferably from 0.000055 to 0.00065, particularly preferably from 0.000085 to 0.00065, even more preferably from 0.00015 to 0.00065, and still even more preferably from 0.00020 to 0.00065, from the viewpoint of interaction between polyaspartic acid alkali metal salts, friction between powder beds, and the like.

(Inorganic Powder)

- Inorganic Powder -

The inorganic powder contained in the powder cosmetic according to the disclosure is not particularly limited, and an inorganic powder generally used as an inorganic powder for powder cosmetics can be used. The shape of the inorganic powder may be a spherical shape, a plate shape, a needle shape, or the like, and may be a porous shape or a non-porous shape.

The material of the inorganic powder may be any of general inorganic powder, bright powder, organic powder, pigment powder, composite powder, or the like. Specific examples of the inorganic powder include: a general inorganic powder such as titanium oxide, black titanium oxide, Prussian blue, ultramarine blue, red iron oxide, yellow iron oxide, black iron oxide, zinc oxide, aluminum oxide, silica, magnesium oxide, zirconium oxide, magnesium carbonate, calcium carbonate, chromium oxide, chromium hydroxide, carbon black, aluminum silicate, magnesium silicate, aluminum magnesium silicate, mica, synthetic mica, synthetic mica iron, sericite, talc, kaolin, silicon carbide, barium sulfate, bentonite, smectite, or boron nitride; a bright inorganic powder such as bismuth oxychloride, titanium oxide-coated mica, iron oxide-coated mica, iron oxide-coated mica titanium, iron oxide-titanium oxide sintered body, or aluminum powder; and a composite inorganic powder such as fine particle titanium oxide-coated mica titanium, fine particle zinc oxide-coated mica titanium, barium sulfate-coated mica titanium, titanium oxide-containing silica, or zinc oxide-containing silica. As the powder cosmetic according to the disclosure, any one of these inorganic powders may be used singly, or two or more thereof may be contained in any combination.

The inorganic powder according to the disclosure preferably contains at least one selected from the group consisting of talc, mica, sericite, and titanium oxide, from the viewpoint of having smooth spreadability with less resistance feeling to the skin and suppressing occurrence of makeup creasing at the time of overlaying the powder cosmetic. The inorganic powder according to the disclosure may be, for example, a mixture of talc and titanium oxide. The inorganic powder according to the disclosure more preferably contains at least one selected from the group consisting of talc, mica, and sericite, and still more preferably contains talc.

- Average Particle Diameter -

The particle diameter of the inorganic powder contained in the powder cosmetic according to the disclosure is not particularly limited. An appropriate particle diameter of the inorganic powder can be selected according to the purpose in a range from an ultrafine particle size of about 0.02 $\mu$m to a relatively large size of about 200 $\mu$m in terms of the average particle diameter. The average particle diameter according to the disclosure refers to a value of a particle diameter at which the integrated volume is 50% of the total volume when the volumes of respective particles based on the particle diameters of respective particles are integrated from the small particle diameter side, the particle diameters being measured in a state where an inorganic powder is dispersed in a solvent. That is, the average particle diameter according to the disclosure has the same meaning as the median diameter, and is also referred to as "average particle diameter (D50)" or "volume average particle diameter D50". The volume average particle diameter D50 of the inorganic powder contained in the powder cosmetic according to the disclosure can be measured by the following procedure. An inorganic powder is dispersed in hexane (special grade chemical, solvent refractive index: 1.38) (manufactured by FUJIFILM Wako Pure Chemical Corporation) used as a dispersing solvent in circulation in an apparatus. Measurement is performed using Microtrac MT3300EXII (particle size distribution meter, manufactured by MicrotracBEL Corp.) at a circulation speed of 50% (65 mL/sec at 100%) under concentration condition within an appropriate amount range of the Loading Index of the apparatus.

The average particle diameter of the inorganic powder according to the disclosure is not particularly limited, and is, for example, from 1 $\mu$m to 100 $\mu$m. The average particle diameter of the inorganic powder is preferably from 3 $\mu$m to 60 $\mu$m, more preferably from 4 $\mu$m to 40 $\mu$m, still more preferably from 5 $\mu$m to 30 $\mu$m, and particularly preferably from 6 $\mu$m to 20 $\mu$m, from the viewpoint that when the average particle diameter is too small, sense of use cannot be obtained, and when the average particle diameter is too large, sense of use deteriorates.

(Other Powders)

The powder cosmetic according to the disclosure may contain other powders other than the inorganic powder whose surface is partially or entirely coated with the polyaspartic acid alkali metal salt as long as the effects thereof are not impaired. Examples of the other powders include: the inorganic powders exemplified above; an organic powder such as organic pigment-coated mica titanium, nylon powder, polymethyl methacrylate powder, acrylonitrile-methacrylic acid copolymer powder, vinylidene chloride-methacrylic acid copolymer powder, polyethylene powder, polystyrene powder, organopolysiloxane elastomer powder, polymethylsilsesquioxane powder, polyurethane powder, wool powder, silk powder, or crystalline cellulose powder; and a dye powder such as an organic tar-based pigment or a lake pigment of an organic dye, and the surfaces of these powders are not coated with the polyaspartic acid alkali metal salt. The powder cosmetic according to the disclosure may contain additional components described in the section of the powder cosmetic composition described later as long as the effects thereof are not impaired.

<<Method of Producing Powder Cosmetic>>

The method of producing a powder cosmetic according to the disclosure may include: mixing an inorganic powder with a polyaspartic acid alkali metal salt to obtain a mixture containing the inorganic powder and the polyaspartic acid alkali metal salt; pulverizing the mixture; and drying the pulverized mixture.

The powder cosmetic according to the disclosure can be produced by the production method. However, the method of producing a powder cosmetic according to the disclosure is not limited to the above-described production method, and the powder cosmetic according to the disclosure can be produced by another method.

Mixing of the inorganic powder and the polyaspartic acid alkali metal salt may be performed at room temperature, for example, but is not limited thereto. Mixing may be performed at any temperature at which the aqueous solution of the polyaspartic acid alkali metal salt does not solidify or boil. For example, mixing may be performed at a temperature within a temperature range of from 5°C to 95°C. At the time of mixing, an aqueous medium may coexist, but the aqueous medium does not necessarily exist. That is, mixing the inorganic powder with the polyaspartic acid alkali metal salt may or need not include mixing the inorganic powder, the polyaspartic acid alkali metal salt, and the aqueous medium. The aqueous medium may be simply water or a mixed solvent of water and a water-miscible solvent. Examples of such a water-miscible solvent include alcohols such as methanol, ethanol, propanol, and ethylene glycol, and acetone.

The inorganic powder and the polyaspartic acid alkali metal salt can be mixed using a disper, a Henschel mixer, a Loedige mixer, a kneader, a V-type mixer, a roll mill, a bead mill, a twin-screw kneader, or the like. The mixing time may be, for example, from 1 minute to 1 hour, or may be from 2 minutes to 20 minutes. Mixing may be performed, for example, by adding the polyaspartic acid alkali metal salt or an aqueous solution of the polyaspartic acid alkali metal salt to the inorganic powder or a mixed solution containing the inorganic powder and the aqueous medium. In this case, the entire amount of the polyaspartic acid alkali metal salt may be added from the beginning, or a partial amount thereof may be added in several times.

The addition amount of the polyaspartic acid alkali metal salt may be set to be from 0.00000095 to 0.0045 or from 0.000035 to 0.0010 with respect to the total amount of the inorganic powder and the polyaspartic acid alkali metal salt. By setting the addition amount of the polyaspartic acid alkali metal salt to be from 0.00000095 to 0.0045 with respect to the total amount of the inorganic powder and the polyaspartic acid alkali metal salt, it is possible to obtain a powder cosmetic that has even more smooth spreadability with less resistance feeling to the skin and is less likely to cause makeup creasing. When the inorganic powder and the polyaspartic acid alkali metal salt are mixed, the other powders described above, additional components described in the section of the powder cosmetic composition described later, and the like may be mixed together in addition to the inorganic powder and the polyaspartic acid alkali metal salt.

In the method of producing a powder cosmetic according to the disclosure, the description of the type and the weight average molecular weight of the polyaspartic acid alkali metal salt in the powder cosmetic according to the disclosure described above can be used for the description of the type and the weight average molecular weight of the polyaspartic acid alkali metal salt. In the method of producing a powder cosmetic according to the disclosure, the above-described description of the expressions satisfied by the weight average molecular weight x of the polyaspartic acid alkali metal salt and the mass ratio y of the polyaspartic acid alkali metal salt to the total amount of the inorganic powder and the polyaspartic acid alkali metal salt in the powder cosmetic according to the disclosure can be used for the description of the expressions satisfied by the weight average molecular weight x of the polyaspartic acid alkali metal salt and the mass ratio y of the polyaspartic acid alkali metal salt to the total amount of the inorganic powder and the polyaspartic acid alkali metal salt.

The mixture that is obtained by the mixing and that contains the inorganic powder and the polyaspartic acid alkali metal salt, or the mixed solution containing an aqueous medium in addition to the mixture is subjected to a pulverization treatment. The pulverization treatment can be performed using a jaw crusher, a gyratory crusher, an impact crusher, a cone crusher, a roll crusher, a cutter mill, an autogenous pulverizer, a stamp mill, a stone mill type, a mortar, a grinding machine, a ring mill, a roller mill, a jet mill, a hammer mill, a pin mill, a rotary mill, a vibration mill, a planetary mill, an attritor, a bead mill, an atomizer, or the like. A suitable pulverizer may be selected so as to obtain a desired particle diameter according to the use of the powder cosmetic.

The mixture after the pulverization treatment may be optionally filtered, if necessary. An inorganic powder having a desired particle diameter can be recovered by filtration, and in a case in which an aqueous medium is present, drying can be facilitated by separating the inorganic powder from the aqueous medium. The pore size of the filter used for filtration can be selected according to a desired particle diameter.

A powder cosmetic containing an inorganic powder whose surface is partially or entirely coated with the polyaspartic acid alkali metal salt is obtained by drying a mixture in the mixed solution after the pulverization treatment or by drying the inorganic powder obtained by the optionally performed filtration. The drying method is not particularly limited. Drying may be performed by natural drying, under reduced pressure, or under heating. In the case of reducing the pressure, for example, the pressure may be reduced to about 1 kPa to 20 kPa in absolute pressure, and in the case of heating, for example, the drying may be performed by heating to about 60°C to 150°C.

The inorganic powder that is obtained by drying and that has a surface partially or entirely coated with the polyaspartic acid alkali metal salt may be optionally mixed with the other powders described above.

<<Cosmetic Composition>>

The powder cosmetic according to the disclosure may be used singly as a powder, but may be used in the form of a cosmetic composition containing the powder cosmetic according to the disclosure and at least one selected from water or an oil agent. That is, the cosmetic composition according to the disclosure may be a cosmetic composition containing the powder cosmetic according to the disclosure, and at least one selected from water or an oil agent. The cosmetic composition according to the disclosure has an effect of having smooth spreadability with less resistance feeling to the skin and suppressing occurrence of makeup creasing even when used in such a cosmetic composition.

Examples of the oil agent include: a hydrocarbon such as paraffin wax, ceresin wax, ozokerite, microcrystalline wax, montan wax, Fischer-Tropsch wax, polyethylene wax, liquid paraffin, squalane, vaseline, polyisobutylene, or polybutene; a natural wax such as carnauba wax, beeswax, lanolin wax, or candelilla; an ester such as glyceryl tribehenate, pentaerythritol rosinate, jojoba oil, cetyl isooctanate, isopropyl myristate, glyceryl trioctanoate, diglyceryl triisostearate, or dipentaerythritol fatty acid ester; a fatty acid such as stearic acid, behenic acid, or 12-hydroxystearic acid; a higher alcohol such as cetanol, stearyl alcohol, or behenyl alcohol; an oil and fat such as olive oil, castor oil, mink oil, or Japan wax; a lanolin derivative such as lanolin fatty acid isopropyl or lanolin alcohol; an amino acid derivative such as N-lauroyl-L-glutamic acid di(cholesteryl/behenyl/octyldodecyl); and a fluorine-based oil agent such as perfluoropolyether, perfluorodecane, or perfluorooctane.

The cosmetic composition according to the disclosure may contain components other than the powder cosmetic, water, and the oil agent as long as the effects thereof are not impaired. Examples of such additional components include components usually used in cosmetics. The component is, for example, a water-soluble component such as a water-miscible solvent, a surfactant, an oil gelling agent, a polyhydric alcohol or a moisturizer, an ultraviolet absorber, a preservative, a cosmetic component, or a fragrance.

The cosmetic composition according to the disclosure can be produced by mixing the powder cosmetic according to the disclosure with at least one selected from water or an oil agent, and optionally the other components described above.

The form of the powder cosmetic according to the disclosure and the cosmetic composition according to the disclosure (product form of the cosmetic) is not particularly limited, and can be appropriately selected according to the purpose. The powder cosmetic according to the disclosure or the cosmetic composition according to the disclosure may be, for example, powder, powder pact, two-way cake, powder foundation, liquid foundation, cream foundation, concealer, BB cream, CC cream, foundation primer, primer, eye shadow, or blusher.

As described above, the powder cosmetic and the cosmetic composition according to the disclosure exhibit an effect of having smooth spreadability with less resistance feeling to the skin and suppressing occurrence of makeup creasing.

Examples

Hereinafter, the disclosure will be described more specifically with reference to examples, but the disclosure is not limited to the following examples unless it goes beyond the gist of the disclosure. Unless otherwise specified, the unit "parts" are on a mass basis. The unit "%" is also on a mass basis.

<Example 1>

An aqueous solution of sodium polyaspartate A was prepared as follows. First, 320 g of L-aspartic acid (special grade chemical, manufactured by FUJIFILM Wako Pure Chemical Corporation) was spread on a SUS tray. The tray was allowed to stand in an oven at 230°C for 4 hours under normal pressure in a nitrogen atmosphere to obtain 233 g of polysuccinimide as a powder. Then, 166 g of distilled water was added to 233 g of the obtained polysuccinimide, and a 32 mass% aqueous solution of sodium hydroxide was added dropwise little by little while the temperature of the solution was controlled to be from 45°C to 55°C. When the reactant acquired fluidity, pH measurement was started. The 32 mass% aqueous solution of sodium hydroxide was further added dropwise to the solution while the pH of the solution was measured. The dropwise addition was terminated when the pH fell within a range of from 10 to 10.5. The amount of the 32 mass% aqueous solution of sodium hydroxide used was 291 g. As a result, a uniform brown transparent aqueous solution of sodium polyaspartate A was obtained. Furthermore, 128 g of distilled water was added to the aqueous solution, to obtain 818 g of a 40 mass% aqueous solution of sodium polyaspartate A.

The molecular weight of the sodium polyaspartate A contained in the obtained 40 mass% aqueous solution of sodium polyaspartate A was measured. The weight average molecular weight (Mw) was 22,000, and the number average molecular weight (Mn) was 14,000. The molecular weight was measured by the apparatus of gel permeation chromatography (GPC) and the GPC measurement described above.

As the inorganic powder, 1.0 kg of talc (JA-46R (for cosmetics), manufactured by Asada Milling Co., Ltd.) was charged into a Henschel mixer (model number FM10C/I: manufactured by Nippon Coke & Engineering. Co., Ltd.). The 40 mass% aqueous solution of sodium polyaspartate A was further used, and the 40 mass% aqueous solution of sodium polyaspartate A diluted to 10 g with distilled water was added so that the addition amount of the sodium polyaspartate A with respect to the inorganic powder was a predetermined amount (the mass ratio of the sodium polyaspartate A to the total amount of the inorganic powder and the sodium polyaspartate A is 0.0004) shown in Table 1. The addition was temporarily stopped at a time when half of a predetermined amount of the 40 mass% aqueous solution of sodium polyaspartate A was added, and mixing was performed by the Henschel mixer at 3,000 rpm (rotation/min) for 3 minutes. Furthermore, the remaining half of the predetermined amount of the 40 mass% aqueous solution of sodium polyaspartate A was added, and mixing was performed by the Henschel mixer at 3,000 rpm (rotation/min) for 3 minutes.

The powder after mixing by the Henschel mixer was subjected to an atomizer (model number K2-1, manufactured by Fuji Paudal Co, Ltd.) to pulverize the solid content. Pulverization with the atomizer was performed with a screen diameter of 2 mmφ and a screw pitch of 20 mm using a stepped liner. The pulverization time was from 30 minutes to 60 minutes. The obtained pulverized product was filtered with a filter cloth having a pore size of 2 mm, and the powder on the filter cloth was recovered.

The recovered powder was dried in a tool dryer. For the drying temperature and the drying time, drying was performed at 80°C for 3 hours and then at 40°C overnight. As a result of drying, an inorganic powder (hereinafter, also referred to as "mixed powder") whose surface was partially or entirely coated with sodium polyaspartate A was obtained.

<Examples 2 to 5 and Comparative Example 2>

In Examples 2 to 5 and Comparative Example 2, mixed powders were obtained in the same manner as in Example 1 except that the mass ratio of the sodium polyaspartate A to the total amount of the inorganic powder and the sodium polyaspartate A was changed as shown in Table 1.

<Comparative Example 1>

In Comparative Example 1, a mixed powder was obtained in the same manner as in Example 1 except that 10 g of the 40 mass% aqueous solution of sodium polyaspartate A was added to 1.0 kg of the inorganic powder, and the mass ratio of the sodium polyaspartate A to the total amount of the inorganic powder and the sodium polyaspartate A was changed to 0.004.

<Example 6>

In Example 6, sodium polyaspartate B obtained as follows was used instead of the sodium polyaspartate A in Example 1. In accordance with the method of Example 1 of JP-B No. 3384420, 133.0 g of L-aspartic acid and 200 g of liquid paraffin (special grade chemical, manufactured by FUJIFILM Wako Pure Chemical Corporation) were charged into a 1 L flask. This was reacted at 220°C for 4 hours and cooled to 50°C. Furthermore, 286.0 g of a 14% aqueous solution of sodium hydroxide was added to the cooled resultant, and the mixture was stirred for 1 hour. The reactant was separated into an aqueous layer and an organic layer, and the aqueous layer was taken out. The obtained aqueous layer was cloudy due to residual liquid paraffin. The aqueous layer was treated by adding Celite and activated carbon to the aqueous layer, thereby obtaining a yellow transparent uniform aqueous solution of sodium polyaspartate B. The molecular weight of the sodium polyaspartate B was measured by GPC measurement. The weight average molecular weight (Mw) was 10,000, and the number average molecular weight (Mn) was 7,000. A mixed powder was obtained in the same manner as in Example 1 except that the sodium polyaspartate B was used.

<Example 7, Example 8, and Comparative Example 4>

In Example 7, Example 8, and Comparative Example 4, mixed powders were obtained in the same manner as in Example 6 except that the mass ratio of the sodium polyaspartate B to the total amount of the inorganic powder and the sodium polyaspartate B was changed as shown in Table 1.

<Comparative Example 3>

In Comparative Example 3, a mixed powder was obtained in the same manner as in Example 6 except that 58.7 g of a 30 mass% aqueous solution of sodium polyaspartate B was added to 1.0 kg of the inorganic powder, and the mass ratio of the sodium polyaspartate B to the total amount of the inorganic powder and the sodium polyaspartate B was changed to 0.0176.

<Example 9>

In Example 9, sodium polyaspartate C obtained as follows was used instead of the sodium polyaspartate A in Example 1.
That is, 118 g of L-aspartic acid (special grade chemical, manufactured by FUJIFILM Wako Pure Chemical Corporation), 235 g of sulfolane (manufactured by FUJIFILM Wako Pure Chemical Corporation, no grade), 83 g of xylene (manufactured by FUJIFILM Wako Pure Chemical Corporation, Wako special grade), and 70 g of 35% hydrochloric acid (special grade chemical, manufactured by FUJIFILM Wako Pure Chemical Corporation) were charged into a 3 L four-necked flask, and reacted at 110°C to 115°C for 3 hours. The distilled water was collected and extracted by a Dean-Stark apparatus, if appropriate. Thereafter, the mixture was reacted at 118°C to 147°C for 5 hours and at 147°C to 154°C for 5 hours. Further, xylene was collected and extracted at 154°C to 162°C for 2 hours by a Dean-Stark apparatus. Thereafter, 1,600 g of acetonitrile (special grade chemical, manufactured by FUJIFILM Wako Pure Chemical Corporation) was added to the resultant, followed by crystallization and stirring at room temperature for 1 hour. Thereafter, filtration was performed with a filter paper having a pore size of 7 μm, and the white powder remaining on the filter paper was dried in a vacuum dryer at 70°C and 5 Torr for 24 hours to obtain 81 g of polysuccinimide as a white powder. Then, 99 g of distilled water was added to 81 g of the obtained polysuccinimide, and a 32 mass% aqueous solution of sodium

**EP 4 197 597 A1**

hydroxide was added dropwise little by little while the temperature of the solution was controlled to be from 45°C to 55°C. When the reactant acquired fluidity, pH measurement was started. The 32 mass% aqueous solution of sodium hydroxide was further added dropwise to the solution while the pH of the solution was measured. The dropwise addition was terminated when the pH fell within a range of from 10 to 10.5. The amount of the 32 mass% aqueous solution of sodium hydroxide used was 94 g. As a result, a yellow transparent uniform aqueous solution of sodium polyaspartate C was obtained. Furthermore, 12 g of distilled water was added to the aqueous solution, to obtain 286 g of a 40 mass% aqueous solution of sodium polyaspartate C. The molecular weight of the obtained sodium polyaspartate C was measured by GPC measurement. The weight average molecular weight (Mw) was 50,000, and the number average molecular weight (Mn) was 30,000.

A mixed powder was obtained in the same manner as in Example 1 except that the sodium polyaspartate C was used and the mass ratio of the sodium polyaspartate C to the total amount of the inorganic powder and the sodium polyaspartate C was changed to 0.00001.

<Example 10, Example 11, and Comparative Examples 5 to 7>

In Example 10, Example 11, and Comparative Examples 5 to 7, mixed powders were obtained in the same manner as in Example 9 except that the mass ratio of the sodium polyaspartate C to the total amount of the inorganic powder and the sodium polyaspartate C was changed as described in Table 1.

<Example 12>

In Example 12, sodium polyaspartate D obtained as follows was used instead of the sodium polyaspartate A in Example 1.

That is, 58 g of L-aspartic acid (special grade chemical, manufactured by FUJIFILM Wako Pure Chemical Corporation), 96 g of sulfolane (manufactured by FUJIFILM Wako Pure Chemical Corporation, no grade), and 29 g of polyphosphoric acid-105 (manufactured by Nippon Chemical Industrial Co., Ltd.) were charged into a 3 L four-necked flask, and reacted at 155°C to 175°C for 3 hours. The distilled water was collected and extracted by a Dean-Stark apparatus, if appropriate. Thereafter, the reactant was diluted with 203 g of N,N-dimethylformamide (special grade chemical, manufactured by FUJIFILM Wako Pure Chemical Corporation) and cooled to 50°C. Furthermore, 348 g of methanol (special grade chemical, manufactured by FUJIFILM Wako Pure Chemical Corporation) was added to the cooled resultant, followed by crystallization and stirring at room temperature for 1 hour. Thereafter, filtration was performed with a filter paper having a pore size of 7 $\mu$m, and the white powder remaining on the filter paper was dried in a vacuum dryer at 100°C and 5 Torr for 24 hours to obtain 43 g of polysuccinimide as a white powder. Then, 51 g of distilled water was added to 43 g of the obtained polysuccinimide, and a 32 mass% aqueous solution of sodium hydroxide was added dropwise little by little while the temperature of the solution was controlled to be from 45°C to 55°C. When the reactant acquired fluidity, pH measurement was started. The 32 mass% aqueous solution of sodium hydroxide was further added dropwise to the solution while the pH of the solution was measured. The dropwise addition was terminated when the pH fell within a range of from 10 to 10.5. The amount of the 32 mass% aqueous solution of sodium hydroxide used was 55 g. As a result, a yellow transparent uniform aqueous solution of sodium polyaspartate D was obtained. Furthermore, 3 g of distilled water was added to the aqueous solution, to obtain 152 g of a 40 mass% aqueous solution of sodium polyaspartate D. The molecular weight of the obtained sodium polyaspartate D was measured by GPC measurement. The weight average molecular weight (Mw) was 120,000, and the number average molecular weight (Mn) was 48,000.

A mixed powder was obtained in the same manner as in Example 1 except that the sodium polyaspartate D was used and the mass ratio of the sodium polyaspartate D to the total amount of the inorganic powder and the sodium polyaspartate D was changed to 0.000001.

<Comparative Examples 8 and 9>

In Comparative Examples 8 and 9, mixed powders were obtained in the same manner as in Example 12 except that the mass ratio of the sodium polyaspartate D to the total amount of the inorganic powder and the sodium polyaspartate D was changed as described in Table 1.

<Example 13>

In Example 13, a mixed powder was obtained in the same manner as in Example 1 except that sericite (OC-100R, manufactured by Ohchem Commerce Co., Ltd.) was used as the inorganic powder.

15

<Comparative Example 10>

In Comparative Example 10, a mixed powder was obtained in the same manner as in Example 13 except that 10 g of the 40 mass% aqueous solution of sodium polyaspartate A was added to 1.0 kg of the inorganic powder, and the mass ratio of the sodium polyaspartate A to the total amount of the inorganic powder and the sodium polyaspartate A was changed to 0.004.

<Example 14>

In Example 14, a mixed powder was obtained in the same manner as in Example 1 except that mica (Y-2300X, manufactured by Yamaguchi Mica Co., Ltd.) was used as the inorganic powder, and the mass ratio of the sodium polyaspartate A to the total amount of inorganic powder and the sodium polyaspartate A was changed to 0.0001.

<Measurement of Powder Bed Shear Force>

For the inorganic powders (mixed powders) that were obtained in Examples 1 to 14 and Comparative Examples 1 to 10 and that had a surface partially or entirely coated with the polyaspartic acid alkali metal salt, the powder bed shear forces were measured as described below, and the yield loci of the powder beds were obtained.

(Method of Measuring Powder Bed Shear Force)

As an apparatus for measuring the powder bed shear force, a powder bed shear force measuring apparatus, model NS-S500 (manufactured by Nano Seeds Corporation) was used.
The method of measuring the powder bed shear force was as follows.

(1) A powder sample was filled inside a cylindrical cell (upper fixed cell, lower movable cell) and a normal stress was gradually applied to the cells at a constant rate.
(2) After the normal stress reached 150 N, the load of the normal stress was stopped, and relaxation time of the powder bed was provided in a constant volume state.
(3) After sufficient stress relaxation occurred, a horizontal external force was applied to the cells at a constant rate.
(4) After the shear reached a steady state (that is, a state where the values of the normal stress and the shear stress were each constant), the normal stress was gradually attenuated while the horizontal external force was maintained.
(5) The shear stresses $\tau$ (vertical axis; y axis)) corresponding to each normal stress $\sigma$ (horizontal axis; x axis), obtained by detecting the normal stress and the shear stress in the attenuating process, were plotted, and the yield locus was obtained.

More detailed measurement conditions of the powder bed shear force were as follows.

Amount of sample: 20 g
Sampling frequency: 10 Hz
Apparatus configuration identification symbol: BF22XB
Inner diameter of upper fixed cell: 43 mm
Depth of lower movable cell: 5 mm
Shear rate: 10 pm/sec
Thickness of shear band: 0.2 mm
Compression rate: 0.2 mm/sec

In the item (2) of the method of measuring the powder bed shear force, the normal stress was set to 150 N on the assumption of the pressure to be applied to the skin when the powder cosmetic is applied to the skin.
The degree of curves were obtained from the yield loci obtained from respective mixed powders. The degree of curve was calculated from the following equation: degree of curve = [slope of lower part of straight line of yield locus]/[slope of upper part of straight line of yield locus]. Note that the slope of the lower part of the straight line of the yield locus indicates the slope of the straight line of the yield locus when the value of the normal stress $\sigma$ is from 0% to 30%, where the normal stress $\sigma$ when the normal stress $\sigma$ and the shear stress $\tau$ are in a steady state is 100%. The slope of the upper part of the straight line of the yield locus indicates the slope of the straight line of the yield locus when the value of the normal stress $\sigma$ is from 70% to 100%, where the normal stress $\sigma$ when the normal stress $\sigma$ and the shear stress $\tau$ are in a steady state is 100%.
The relative values [-] of the degree of curve of the respective mixed powders in Examples 1 to 14 and Comparative Examples 1 to 10 when the value of the degree of curve of only the inorganic powder is 1.00 are shown in Table 1. In Table 1, the relative value of the degree of curve, the mass ratio of the polyaspartic acid alkali metal salt, and the weight average molecular weight of the polyaspartic acid alkali metal salt have no unit, and thus the units thereof are represented by [-]. In Table 1, the mass ratio of the polyaspartic acid alkali metal salt refers to the mass ratio of the polyaspartic acid alkali metal salt to the total amount of the inorganic powder and the polyaspartic acid alkali metal salt.

A mixed powder having a relative value of the degree of curve of more than 1.00 indicates that the smoothness is decreased as compared with the inorganic powder before addition of the polyaspartic acid alkali metal salt (that is, only the inorganic powder). A mixed powder having a relative value of the degree of curve of less than 1.00 indicates that the smoothness is increased as compared with the inorganic powder before addition of the polyaspartic acid alkali metal salt. When the relative value of the degree of curve of the mixed powder was less than 1.00, it was determined that "the mixed powder has smooth spreadability with less resistance feeling to the skin and is less likely to cause makeup creasing at the time of overlaying the mixed powder".

[Table 1]

| | Type of Polyaspartic Acid Alkali Metal Salt | Weight Average Molecular Weight x of Polyaspartic Acid Alkali Metal Salt [-] | Type of Inorganic Powder | Mass Ratio y of Polyaspartic Acid Alkali Metal Salt [-] | Relative Value of Degree of Curve [-] |
|---|---|---|---|---|---|
| Example 1 | sodium polyaspartate A | 22,000 | talc | 0.0004 | 0.74 |
| Example 2 | sodium polyaspartate A | 22,000 | talc | 0.0006 | 0.75 |
| Example 3 | sodium polyaspartate A | 22,000 | talc | 0.0008 | 0.84 |
| Example 4 | sodium polyaspartate A | 22,000 | talc | 0.0001 | 0.81 |
| Example 5 | sodium polyaspartate A | 22,000 | talc | 0.00004 | 0.89 |
| Comparative Example 1 | sodium polyaspartate A | 22,000 | talc | 0.004 | 1.01 |
| Comparative Example 2 | sodium polyaspartate A | 22,000 | talc | 0.00001 | 1.04 |
| Example 6 | sodium polyaspartate B | 10,000 | talc | 0.0004 | 0.87 |
| Example 7 | sodium polyaspartate B | 10,000 | talc | 0.0006 | 0.8 |
| Example 8 | sodium polyaspartate B | 10,000 | talc | 0.004 | 0.91 |
| Comparative Example 3 | sodium polyaspartate B | 10,000 | talc | 0.0176 | 1.28 |
| Comparative Example 4 | sodium polyaspartate B | 10,000 | talc | 0.0001 | 1.08 |
| Example 9 | sodium polyaspartate C | 50,000 | talc | 0.00001 | 0.87 |
| Example 10 | sodium polyaspartate C | 50,000 | talc | 0.00004 | 0.78 |
| Example 11 | sodium polyaspartate C | 50,000 | talc | 0.0001 | 0.73 |
| Comparative Example 5 | sodium polyaspartate C | 50,000 | talc | 0.0006 | 1.08 |

(continued)

| | Type of Polyaspartic Acid Alkali Metal Salt | Weight Average Molecular Weight x of Polyaspartic Acid Alkali Metal Salt [-] | Type of Inorganic Powder | Mass Ratio y of Polyaspartic Acid Alkali Metal Salt [-] | Relative Value of Degree of Curve [-] |
|---|---|---|---|---|---|
| Comparative Example 6 | sodium polyaspartate C | 50,000 | talc | 0.0004 | 1.06 |
| Comparative Example 7 | sodium polyaspartate C | 50,000 | talc | 0.000001 | 1.09 |
| Example 12 | sodium polyaspartate D | 120,000 | talc | 0.000001 | 0.97 |
| Comparative Example 8 | sodium polyaspartate D | 120,000 | talc | 0.0008 | 1.03 |
| Comparative Example 9 | sodium polyaspartate D | 120,000 | talc | 0.0001 | 1.01 |
| Example 13 | sodium polyaspartate A | 22,000 | sericite | 0.0004 | 0.94 |
| Comparative Example 10 | sodium polyaspartate A | 22,000 | sericite | 0.004 | 1.37 |
| Example 14 | sodium polyaspartate A | 22,000 | mica | 0.0001 | 0.92 |

The relationship of the relative value of the degree of curve with respect to the weight average molecular weight [-] of the polyaspartic acid alkali metal salt and the mass ratio [-] of the polyaspartic acid alkali metal salt in Examples 1 to 14 and Comparative Examples 1 to 10 is shown in Fig. 2. In Fig. 2, the horizontal axis represents the weight average molecular weight of the polyaspartic acid alkali metal salt. The weight average molecular weight is a relative value and has no unit, and thus the unit is represented by [-]. In Fig. 2, the vertical axis represents the mass ratio of the polyaspartic acid alkali metal salt (that is, the mass ratio of the polyaspartic acid alkali metal salt to the total amount of the inorganic powder and the polyaspartic acid alkali metal salt.). The mass ratio of the polyaspartic acid alkali metal salt does not have a unit, and thus the unit is represented by [-]. Note that the vertical axis is represented by a logarithmic scale. In Fig. 2, black circles indicate examples, that is, indicate that the relative value of degree of curve of the mixed powder is less than 1.00. White circles represent comparative examples.

From Table 1 and Fig. 2, it was confirmed that the relative value of the degree of curve depended on the weight average molecular weight [-] of the polyaspartic acid alkali metal salt and the mass ratio of the polyaspartic acid alkali metal salt.

From the above, regarding the weight average molecular weight x of the polyaspartic acid alkali metal salt and the mass ratio y of the polyaspartic acid alkali metal salt to the total amount of the inorganic powder and the polyaspartic acid alkali metal salt, in Examples 1 to 14 which were included in a region surrounded by a range of Expression (1) (or Expression (3) or (4)) and Expression (2) (or Expression (5)) and the weight average molecular weight x of the polyaspartic acid alkali metal salt of from 1,000 to 150,000 (or from 8,000 to 120,000), a powder cosmetic that has smooth spreadability with less resistance feeling to the skin and is less likely to cause makeup creasing at the time of overlaying the powder cosmetic could be obtained. Meanwhile, in Comparative Examples 1 to 10 which were not included in the region surrounded by the above-described range, the powder cosmetic as described above could not be obtained.

The entire disclosure of Japanese Patent Application No. 2020-154913 filed on September, 15, 2020 is incorporated herein by reference.

All publications, patent applications, and technical standards described in the present specification are incorporated herein by reference to the same extent as if each publication, patent application, or technical standard was specifically and individually indicated to be incorporated by reference.

**Claims**

1. A powder cosmetic comprising:

an inorganic powder; and

a polyaspartic acid alkali metal salt that coats a part or all of a surface of the inorganic powder,

wherein a weight average molecular weight x of the polyaspartic acid alkali metal salt is from 1,000 to 150,000, and

wherein the weight average molecular weight x of the polyaspartic acid alkali metal salt and a mass ratio y of the polyaspartic acid alkali metal salt to a total amount of the inorganic powder and the polyaspartic acid alkali metal salt satisfy the following Expressions (1) and (2):

$$y \leq 1.6 \times 10^7 x^{-2.275} \ldots \text{Expression (1)}$$

$$y \geq 1.3 \times 10^5 x^{-2.232} \ldots \text{Expression (2)}.$$

2. The powder cosmetic according to claim 1, wherein the weight average molecular weight x of the polyaspartic acid alkali metal salt and the mass ratio y of the polyaspartic acid alkali metal salt to the total amount of the inorganic powder and the polyaspartic acid alkali metal salt further satisfy the following Expression (3):

$$y \leq 1.0 \times 10^7 x^{-2.275} \ldots \text{Expression (3)}.$$

3. The powder cosmetic according to claim 1, wherein the weight average molecular weight x of the polyaspartic acid alkali metal salt and the mass ratio y of the polyaspartic acid alkali metal salt to the total amount of the inorganic powder and the polyaspartic acid alkali metal salt further satisfy the following Expression (4):

$$y \leq 8.0 \times 10^6 x^{-2.275} \ldots \text{Expression (4)}.$$

4. The powder cosmetic according to any one of claims 1 to 3, wherein the weight average molecular weight x of the polyaspartic acid alkali metal salt and the mass ratio y of the polyaspartic acid alkali metal salt to the total amount of the inorganic powder and the polyaspartic acid alkali metal salt further satisfy the following Expression (5):

$$y \geq 1.5 \times 10^5 x^{-2.232} \ldots \text{Expression (5)}.$$

5. The powder cosmetic according to any one of claims 1 to 4, wherein the weight average molecular weight x of the polyaspartic acid alkali metal salt is from 8,000 to 120,000.

6. The powder cosmetic according to any one of claims 1 to 5, wherein the polyaspartic acid alkali metal salt is at least one selected from the group consisting of lithium polyaspartate, potassium polyaspartate, and sodium polyaspartate.

7. The powder cosmetic according to any one of claims 1 to 6, wherein the polyaspartic acid alkali metal salt contains at least sodium polyaspartate.

8. The powder cosmetic according to any one of claims 1 to 7, wherein the inorganic powder contains at least one selected from the group consisting of talc, mica, sericite, and titanium oxide.

9. The powder cosmetic according to any one of claims 1 to 8, wherein an average particle diameter (D50) of the inorganic powder is from 1 $\mu$m to 100 $\mu$m.

10. A cosmetic composition comprising the powder cosmetic according to any one of claims 1 to 9, and at least one selected from water or an oil agent.

## FIG.1

YIELD LOCUS    STEADY STATE

SHEAR STRESS τ

SLOPE OF UPPER PART
OF STRAIGHT LINE

SLOPE OF LOWER PART
OF STRAIGHT LINE

0%    30%    70%    100%

NORMAL STRESS σ

# FIG.2

## INTERNATIONAL SEARCH REPORT

| International application No. |
|---|
| **PCT/JP2021/032136** |

### A. CLASSIFICATION OF SUBJECT MATTER

*A61Q 1/00*(2006.01)i; *A61K 8/02*(2006.01)i; *A61K 8/25*(2006.01)i; *A61K 8/88*(2006.01)i
FI:    A61K8/25; A61K8/88; A61K8/02; A61Q1/00

According to International Patent Classification (IPC) or to both national classification and IPC

### B. FIELDS SEARCHED

Minimum documentation searched (classification system followed by classification symbols)

A61Q1/00; A61K8/02; A61K8/25; A61K8/88; C09C3/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2007-537116 A (BASF AKTIENGESELLSCHAFT) 20 December 2007 (2007-12-20) claims 1-3, 12-14, paragraphs [0020]-[0021], [0046]-[0047], [0049] | 1-10 |
| X | JP 2006-265214 A (KOSE CORP.) 05 October 2006 (2006-10-05) claims 1, 3-4, paragraphs [0001], [0009], [0014], [0017]-[0018], example 1, inventive article 5, table 1 | 1-10 |
| P, X | JP 2020-147525 A (MITSUI CHEMICALS INC.) 17 September 2020 (2020-09-17) entire text | 1-10 |

☐ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **14 October 2021** | **02 November 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/032136**

| Patent document cited in search report | | | Publication date (day/month/year) | Patent family member(s) | Publication date (day/month/year) |
|---|---|---|---|---|---|
| JP | 2007-537116 | A | 20 December 2007 | US 2007/0218019 A1 claims 1, 10-12, paragraphs [0020]-[0021], [0054]-[0055], [0057]<br>WO 2005/094156 A2<br>EP 1743002 A2 | |
| JP | 2006-265214 | A | 05 October 2006 | (Family: none) | |
| JP | 2020-147525 | A | 17 September 2020 | (Family: none) | |

Form PCT/ISA/210 (patent family annex) (January 2015)

**EP 4 197 597 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006265214 A
- JP 3384420 B
- WO 2011102293 A
- JP 2020154913 A